# EUROPEAN PATENT APPLICATION

(11) **EP 4 796 642 A1**
(43) Date of publication of application: **26.08.2026**
(21) Application number: 24878912.5
(22) Date of filing: 12.10.2024
(51) Int. Cl.: C12N 15/864, C07K 14/005, A61K 35/761

(54) **METHOD FOR PRODUCING RECOMBINANT ADENO-ASSOCIATED VIRAL VECTOR AND PACKAGING/PRODUCTION CELL**

(30) Priority: 16.10.2023 CN 202311340195
(71) Applicant: Shenzhen Eureka Biotechnology Co., Limited, Shenzhen, Guangdong 518000 (CN)
(72) Inventor: XUE, Bofu, Shenzhen, Guangdong 518000 (CN); YANG, Weishan, Shenzhen, Guangdong 518000 (CN); YANG, Yinhui, Shenzhen, Guangdong 518000 (CN); CHEN, Li, Shenzhen, Guangdong 518000 (CN); MA, Mo, Shenzhen, Guangdong 518000 (CN)
(74) Representative: Dehns
(86) International application number: PCT/CN2024/124301
(87) International publication number: WO 2025/082268

(57) **Abstract**

Provided is a method for producing a recombinant adeno-associated virus vector without using adenovirus as a helper virus. The method includes using a nucleic acid sequence encoding adenovirus L4-22K protein and/or a nucleic acid sequence encoding adenovirus L4-33K protein as a helper gene sequence for the production of the recombinant adeno-associated virus vector. A recombinant adeno-associated virus vector packaging/production cell is further provided for producing a recombinant adeno-associated virus vector without using adenovirus as a helper virus. The packaging/production cell includes adenovirus L4-22K protein or a nucleic acid encoding adenovirus L4-22K protein, and/or adenovirus L4-33K protein or a nucleic acid encoding adenovirus L4-33K protein.

## Description

### FIELD

The present disclosure relates to a method and a packaging/production cell for producing a recombinant adeno-associated virus vector. Specifically, the present disclosure relates to the use of a specific gene sequence as a helper gene in the method for producing the recombinant adeno-associated virus vector and in construction of the packaging/production cell of the recombinant adeno-associated virus vector, thereby improving the yield of the recombinant adeno-associated virus vector.

### BACKGROUND

Adeno-associated virus (AAV), belonging to the genus *Dependoparvovirus* in the family *Parvoviridae*, requires the presence of a helper virus (e.g., adenovirus) to complete viral replication. AAV is a small, non-enveloped virus whose genome is composed of single-stranded DNA and has a length of approximately 4.7 kb. The genome includes two gene expression cassettes: Rep and Cap. The Rep gene encodes proteins required for viral replication, while the Cap gene encodes capsid proteins. Flanking the Rep and Cap gene expression cassettes are inverted terminal repeats (ITRs), which act as initiation signals for viral DNA replication. Different capsid proteins determine the serotypes of AAV, such as AAV2, AAV5, AAV8, or AAV9.

Recombinant adeno-associated virus (rAAV) vectors containing an exogenous gene of interest (GOI) can be generated by replacing the Rep and Cap gene expression cassettes in the AAV genome with an exogenous nucleic acid fragment of interest through genetic engineering methods. rAAV vectors are widely used in gene therapy due to their ability to efficiently deliver genes to target cells as well as their low toxicity and low immunogenicity.

Due to the replication defect of AAV, rAAV production requires the presence of a helper virus (e.g., adenovirus, herpesvirus, papillomavirus) capable of providing necessary helper factors to facilitate rAAV replication. Alternatively, in the absence of a helper virus, a helper gene from other viruses and capable of promoting AAV replication and assembly must be supplied to host cells used for rAAV production. Such helper genes are typically from adenovirus (e.g., from adenovirus E1A, E1B, E2A, E4, or VA RNA genes), and may also be from herpes virus, papillomavirus, or other viruses. In practical applications, a transgenic plasmid carrying an exogenous GOI, a plasmid carrying the Rep and Cap genes (RC plasmid), or a helper plasmid carrying helper genes can be introduced into host cells to produce rAAV. HEK293 and its derived cell line are currently the most commonly used cell lines for rAAV production. Since the adenovirus E1A and E1B genes have been stably integrated into their genomes, only an additional helper gene plasmid pHelper containing the adenovirus E4 gene, E2A gene, and VA RNA gene needs to be transfected during viral production.

The L4 gene is part of the adenovirus genome and belongs to the late transcription unit. It mainly encodes proteins that constitute the viral capsid or proteins that participate in capsid assembly. The L4 gene is approximately 3.7 kbp in length and accounts for about 10% of the Ad5 genome. The L4 gene encodes four important proteins: L4-100K, L4-33K, L4-22K, and pVIII. The L4-33K protein plays a role in the transition from early to late gene expression during the viral life cycle. It functions as a splicing factor that affects the processing of viral RNA transcripts, allowing the virus to produce proteins required for assembly of new viral particles (Wu K et al., The adenovirus L4-33K protein regulates both late gene expression patterns and viral DNA packaging. J Virol. 2013; 87(12):6739-6747). In the L4 region of Ad5, the coding region of L4-22K overlaps with that of L4-33K. Similar to the L4-33K protein, the L4-22K protein also participates in the regulation of viral gene expression and is involved in the transition from early to late gene expression during the viral life cycle by modulating the splicing of viral RNA transcripts (Wu K et al., supra).

The rapid increase in rAAV gene therapy products has spawned an enormous demand for large-scale production capacity. However, existing industrial production capacity remains insufficient to meet the needs of future commercialization and widespread patient use. Current helper virus-free production systems can only produce rAAV vectors at relatively low titers, even under optimized cell culture conditions. Therefore, there is an urgent need for a method to increase the yield of rAAV vectors.

### SUMMARY

The present inventors have discovered for the first time that introducing a specific gene from an adenovirus genome into an rAAV production system as a helper gene can significantly increase the yield of rAAV vectors, thereby potentially resolving the production yield bottleneck encountered in the commercial production of rAAV vectors.

In an aspect, the present disclosure provides a method for producing an rAAV vector without using adenovirus as a helper virus. The method includes using a nucleic acid sequence encoding adenovirus L4-22K protein and/or a nucleic acid sequence encoding adenovirus L4-33K protein as a helper gene sequence for the production of the rAAV vector. The adenovirus L4-22K protein may be a wild-type L4-22K protein from the family *Adenoviridae*, or a variant having at least 50%, 60%, 70%, 80%, 85%, 90%, 95%, or 99% sequence identity to the amino acid sequence of a wild-type L4-22K protein from the family *Adenoviridae* and capable of exerting an equivalent or similar effect of improving rAAV yield vector. The adenovirus L4-33K protein may be a wild-type L4-33K protein from the family *Adenoviridae*, or a variant having at least 50%, 60%, 70%, 80%, 85%, 90%, 95%, or 99% sequence identity to the amino acid sequence of a wild-type L4-33K protein from the family *Adenoviridae* and capable of exerting an equivalent or similar effect of improving rAAV vector yield. In an example, the adenovirus is a human adenovirus. In an example, the adenovirus is adenovirus Ad5.

In an example, when using both the nucleic acid sequence encoding adenovirus L4-22K protein and the nucleic acid sequence encoding adenovirus L4-33K protein, the nucleic acid sequence encoding adenovirus L4-22K protein and the nucleic acid sequence encoding adenovirus L4-33K protein form a single nucleic acid sequence. In another example, when using both the nucleic acid sequence encoding adenovirus L4-22K protein and the nucleic acid sequence encoding adenovirus L4-33K protein, the nucleic acid sequence encoding adenovirus L4-22K protein overlaps with the nucleic acid sequence encoding adenovirus L4-33K protein.

In an embodiment, the method for producing the rAAV vector further includes using a plurality of additional helper gene sequences.

In an embodiment, the plurality of additional helper gene sequences include one or more nucleic acid sequences from adenovirus, herpes simplex virus, hepatitis C virus, influenza virus, human herpes virus, bocavirus, or human papillomavirus.

In an embodiment, the plurality of additional helper gene sequences include a nucleic acid sequence from adenovirus E1A, E1B, E2A, E4, or VA RNA gene, or a combination thereof. In an embodiment, the additional helper gene sequence may be a nucleic acid sequence from adenovirus E1A gene, a nucleic acid sequence from adenovirus E1B gene, a nucleic acid sequence from adenovirus E2A gene, a nucleic acid sequence from adenovirus E4 gene, or a nucleic acid sequence from adenovirus VA RNA gene, or a combination thereof. In an embodiment, the nucleic acid sequence from adenovirus E4 gene may be a nucleic acid sequence from adenovirus E4orf1, E4orf2, E4orf3, E4orf3/4, E4orf4, E4orf6, or E4orf6/7 gene, or a combination thereof.

In an embodiment, the plurality of additional helper gene sequences include a nucleic acid sequence from herpes simplex virus (HSV) UL5, UL8, UL12, UL29, UL30, UL42, UL52, ICP0, ICP4, ICP22, ICP34.5, or US11 gene, or a combination thereof. In an example, the herpes simplex virus is herpes simplex virus type 1 (HSV-1).

In an embodiment, the nucleic acid sequence from hepatitis C virus (HCV) may be a nucleic acid sequence from hepatitis C virus NS5A gene.

In an embodiment, the nucleic acid sequence from influenza virus may be a nucleic acid sequence from influenza virus NS1 gene.

In an embodiment, the nucleic acid sequence from human herpes virus (HHV) may be a nucleic acid sequence from human herpes virus Rep gene. In an example, the human herpes virus is human herpes virus type 6 (HHV-6).

In an embodiment, the nucleic acid sequence from bocavirus (HBoV) may be a nucleic acid sequence from bocavirus NS2, NS4, NP1, or BocaSR gene, or a combination thereof. In an example, the bocavirus is bocavirus type I (HBoV-1).

In an embodiment, the nucleic acid sequence from human papillomavirus (HPV) may be a nucleic acid sequence from the HPV E1, E2, or E6 gene, or a combination thereof. In an example, the HPV is HPV type 16 (HPV-16).

In an embodiment, the method for producing the rAAV vector includes introducing the nucleic acid sequence encoding adenovirus L4-22K protein and/or the nucleic acid sequence encoding adenovirus L4-33K protein into a host cell.

In an example, the method for producing the rAAV vector includes introducing a vector (e.g., a plasmid) carrying the nucleic acid sequence encoding adenovirus L4-22K protein and/or a vector (e.g., a plasmid) carrying the nucleic acid sequence encoding adenovirus L4-33K protein into a host cell. In an example, the method for producing the rAAV vector includes introducing a vector (e.g., a plasmid) carrying both the nucleic acid sequence encoding adenovirus L4-22K protein and the nucleic acid sequence encoding adenovirus L4-33K protein into the host cell.

In an embodiment, the host cell is a mammalian cell. In an embodiment, the mammalian cell may be selected from HEK293 cells, A549 cells, HeLa cells, HepG2 cells, BHK cells, COS cells, Vero cells, MDCK cells, Per.C6 cells, CAP cells, and a derivative thereof.

In another aspect, the present disclosure provides a recombinant adeno-associated virus vector packaging/production cell for producing a recombinant adeno-associated virus vector without using adenovirus as a helper virus. The packaging/production cell includes: adenovirus L4-22K protein or a nucleic acid encoding adenovirus L4-22K protein, and/or adenovirus L4-33K protein or a nucleic acid encoding adenovirus L4-33K protein. The adenovirus L4-22K protein may be a wild-type L4-22K protein from the Family *Adenoviridae*, or a variant having at least 50%, 60%, 70%, 80%, 85%, 90%, 95%, or 99% sequence identity to the amino acid sequence of a wild-type L4-22K protein from the Family *Adenoviridae* and capable of exerting an equivalent or similar effect of improving rAAV vector yield. The adenovirus L4-33K protein may be a wild-type L4-33K protein from the Family *Adenoviridae*, or a variant having at least 50%, 60%, 70%, 80%, 85%, 90%, 95%, or 99% sequence identity to the amino acid sequence of the wild-type L4-33K protein from the Family *Adenoviridae* and capable of exerting an equivalent or similar effect of improving rAAV vector yield. In an example, the adenovirus is a human adenovirus. In an example, the adenovirus is adenovirus Ad5.

In an embodiment, the packaging/production cell further includes a plurality of additional helper gene sequences. In an embodiment, the plurality of additional helper gene sequences include one or more nucleic acid sequences from adenovirus, herpes simplex virus, hepatitis C virus, influenza virus, human herpes virus, bocavirus, or human papillomavirus.

In an embodiment, the packaging/production cell further includes a nucleic acid sequence from adenovirus E1A, E1B, E2A, E4, or VA RNA gene, or a combination thereof. In an embodiment, the packaging/production cell further includes a nucleic acid sequence from adenovirus E1A gene, a nucleic acid sequence from adenovirus E1B gene, a nucleic acid sequence from adenovirus E2A gene, a nucleic acid sequence from adenovirus E4 gene, a nucleic acid sequence from adenovirus VA RNA gene, or a combination thereof. In an embodiment, the nucleic acid sequence from adenovirus E4 gene may be a nucleic acid sequence from adenovirus E4orf1, E4orf2, E4orf3, E4orf3/4, E4orf4, E4orf6, or E4orf6/7 gene, or a combination thereof.

In an embodiment, the packaging/production cell further includes a nucleic acid sequence from herpes simplex virus UL5, UL8, UL12, UL29, UL30, UL42, UL52, ICP0, ICP4, ICP22, ICP34.5, or US11 gene, or a combination thereof. In an example, the herpes simplex virus is herpes simplex virus type 1.

In an embodiment, the packaging/production cell further includes a nucleic acid sequence from hepatitis C virus NS5A gene.

In an embodiment, the packaging/production cell further includes a nucleic acid sequence from influenza virus NS1 gene.

In an embodiment, the packaging/production cell further includes a nucleic acid sequence from human herpes virus Rep gene. In an example, the human herpes virus is human herpes virus type 6.

In an embodiment, the packaging/production cell further includes a nucleic acid sequence from bocavirus NS2, NS4, NP1, or BocaSR gene, or a combination thereof. In an example, the bocavirus is bocavirus type 1.

In an embodiment, the packaging/production cell further includes a nucleic acid sequence from human papillomavirus (HPV) E1, E2, or E6 gene, or a combination thereof. In an example, the HPV is HPV type 16.

In another aspect, the present disclosure provides use of adenovirus L4-22K protein or a nucleic acid encoding adenovirus L4-22K protein, and/or adenovirus L4-33K protein or a nucleic acid encoding adenovirus L4-33K protein for the production of a recombinant adeno-associated virus vector without using adenovirus as a helper virus.

The present disclosure significantly improves the yield of the method for producing the rAAV vector by using the nucleic acid sequence encoding adenovirus L4-22K protein and/or the nucleic acid sequence encoding adenovirus L4-33K protein as a helper gene sequence for the production of the rAAV vector.

### BRIEF DESCRIPTION OF THE DRAWINGS

FIG. 1 shows the effect of introducing the L4-22K and/or L4-33K gene as a helper gene on rAAV vector production an according to an embodiment of the present disclosure.
FIG. 2 shows the effect of introducing the L4-22K and/or L4-33K gene as a helper gene on rAAV vector production according to another embodiment of the present disclosure.

### DETAILED DESCRIPTION

Embodiments of the present disclosure will be described in detail below. The embodiments described below are exemplary and are only used to explain the present disclosure, and they should not be construed as limiting the present disclosure.

### Example 1: Plasmid Construction Method

The molecular cloning techniques used in the following examples, such as amplification of DNA fragments, restriction endonuclease digestion of DNA fragments, gel recovery of DNA fragments, ligation of two DNA fragments, transformation of competent cells with ligation products, as well as plasmid extraction, preparation and identification, are all well-known and mature techniques in the art. The following reagents were used in the examples: high-fidelity DNA polymerase (Accurate Biology), restriction endonucleases (NEB), DNA fragment gel recovery kit (Omega, D2500-02), plasmid miniprep kit (OMEGA, D6943-02), and chemically competent cells (XL-10 gold). Sequence synthesis was performed by Nanjing Genscript Biotech Co., Ltd. Plasmid sequencing was performed by Tsingke Biotechnology Co., Ltd. Table 1 provides descriptions of SEQ ID NO: 1 to SEQ ID NO: 13. The sequence information of the elements used in the plasmids involved in the following examples was provided for illustrative purposes to implement the present disclosure. It will be appreciated by those skilled in the art that replacing the element sequences on the plasmids used in the following examples with other biologically equivalent element sequences can also achieve the effects described in the present disclosure. Such replacement includes, but not limited to, plasmid backbone sequences (such as origins of replication and resistance genes), restriction enzyme site sequences, transposon repeat sequences, inducible system response element sequence, insulator sequences, promoter sequences, intron sequences, polyadenylation signal (PolyA) sequences, gene sequences with different codon optimizations, mutants of the above-described functional element sequences and gene sequences, as well as the cloning position, cloning order, and cloning orientation of each functional element sequences and gene sequences. The specific plasmid construction method is as follows:
1. Construction of Plasmid pCMV-L4-22K: Using pAdEasy-1 (Addgene, #16400) as a template, the nucleic acid sequence encoding adenovirus L4-22K (SEQ ID NO: 1) was amplified using primers. The amplified fragment was ligated into plasmid pCMV-M1 (Addgene, #23007), thereby generating plasmid pCMV-L4-22K.
2. Construction of Plasmid pCMV-L4-33K: Using pAdEasy-1 (Addgene, #16400) as a template, the nucleic acid sequence encoding adenovirus L4-33K (SEQ ID NO: 3) was amplified using primers. The amplified fragment was ligated into plasmid pCMV-M1 (Addgene, #23007), thereby generating plasmid pCMV-L4-33K.
3. Construction of Plasmid pCMV-L4-22_33K: Using pAdEasy-1 (Addgene, #16400) as a template, the nucleic acid sequence encoding adenovirus L4-22_33K (the L4-22K gene sequence overlapped with the L4-33K gene sequence, SEQ ID NO: 5) was amplified using primers. The amplified fragment was ligated into plasmid pCMV-M1 (Addgene, #23007), thereby generating plasmid pCMV-L4-22_33K.
4. Construction of Plasmid pCMV-UL5: The synthesized nucleic acid sequence fragment encoding HSV-1 UL5 (SEQ ID NO: 6) was ligated into plasmid pCMV-M1 (Addgene, #23007), thereby generating plasmid pCMV-UL5.
5. Construction of Plasmid pCMV-UL8: The synthesized nucleic acid sequence fragment encoding HSV-1 virus UL8 (SEQ ID NO: 8) was ligated into plasmid pCMV-M1 (Addgene, #23007), thereby generating plasmid pCMV-UL8.
6. Construction of Plasmid pCMV-UL29: The synthesized nucleic acid sequence fragment encoding HSV-1 virus UL29 (SEQ ID NO: 10) was ligated into plasmid pCMV-M1 (Addgene, #23007), thereby generating plasmid pCMV-UL29.
7. Construction of Plasmid pCMV-UL52: The synthesized nucleic acid sequence fragment encoding HSV-1 virus UL52 (SEQ ID NO: 12) was ligated into plasmid pCMV-M1 (Addgene, #23007), thereby generating plasmid pCMV-UL52.

**Table 1: Sequence Description**

| SEQ ID NO: | Description |
|---|---|
| 1 | L4-22K nucleic acid sequence (585 bp) |
| 2 | L4-22K protein amino acid sequence (194 aa) |
| 3 | L4-33K nucleic acid sequence (684 bp) |
| 4 | L4-33K protein amino acid sequence (227 aa) |
| 5 | L4-22_33K nucleic acid sequence (886 bp) |
| 6 | UL5 nucleic acid sequence (2649 bp) |
| 7 | UL5 amino acid sequence (882 aa) |
| 8 | UL8 nucleic acid sequence (2253 bp) |
| 9 | UL8 amino acid sequence (750 aa) |
| 10 | UL29 nucleic acid sequence (3591 bp) |
| 11 | UL29 amino acid sequence (1196 aa) |
| 12 | UL52 nucleic acid sequence (3177 bp) |
| 13 | UL52 amino acid sequence (1058 aa) |

### Example 2: Effect of Introducing L4-22K and/or L4-33K Gene as a Helper Gene on rAAV Vector Production When Using Commonly Used Adenovirus Helper Gene

293T cells (ATCC, CRL-3216) were seeded into 6-well plates (Corning 3516) at a density of 8E+05 cells per well in 2 mL of DMEM complete medium. After 24 hours of culture, transfection was performed using the PEI method. During transfection, 200 µl of transfection reagent was added to each well, with a total plasmid amount of 5 µg, including 0.5 µg of pAAV-EF1a-FLuc-WPRE-HGHpA plasmid (AAV transfer vector carrying a firefly luciferase coding sequence under the control of the EF1a promoter and a WPRE detection sequence, flanked by AAV ITR sequences; Addgene#87951, hereinafter referred to as "pAAV-Fluc plasmid"), 0.5 µg of pAAV-RC plasmid (carrying adenovirus Rep and Cap gene sequences; Novopro, V009666#), and 35 ng of pHelper plasmid (carrying adenovirus E4, E2A, and VA RNA gene sequences; Novopro, V005569#). In addition, the Empty group was supplemented with 0.6 µg of empty vector (pCMV-M1 plasmid), the L4-22K group was supplemented with 0.6 µg of pCMV-L4-22K plasmid, the L4-33K group was supplemented with 0.6 µg of pCMV-L4-33K plasmid, the L4-22K+L4-33K group was supplemented with a total of 0.6 µg of pCMV-L4-22K and pCMV-L4-33K plasmids (0.3 µg each), and the L4-22_33K group was supplemented with 0.3 µg of pCMV-L4-22_33K plasmid. The remaining plasmid amount was made up with the above-described empty vector. The mass ratio of total plasmid amount to PEI was 1:4, and the experiment was independently repeated twice. 48 hours after cell transfection, an equal volume of 1% Tween 20 was added for cell lysis. After the cell lysate was treated with a non-specific nuclease (Yeasen Biotechnology (Shanghai) Co., Ltd., 20156ES60), the adeno-associated virus genome (vg) copy numbers were measured using an ABI QuantStudio 5 real-time fluorescent quantitative PCR system.

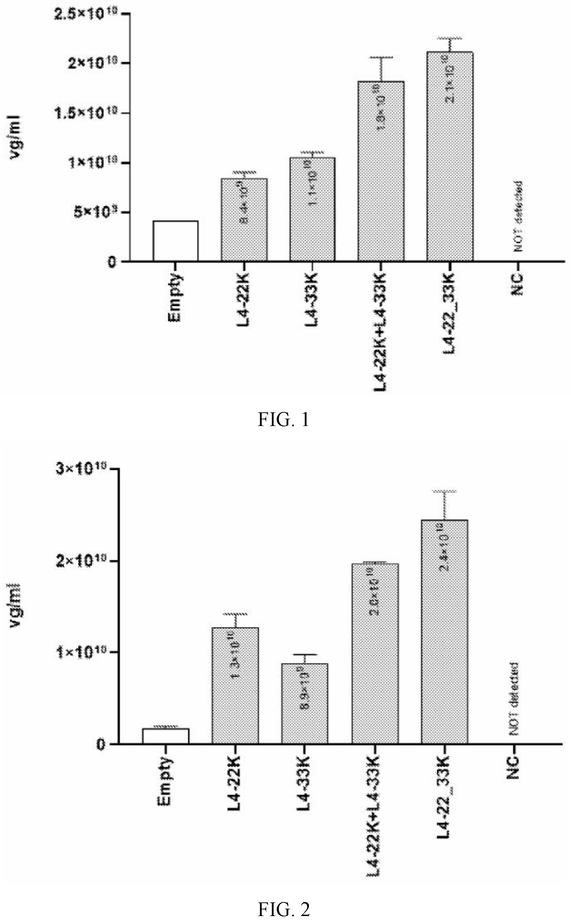

In FIG. 1: Empty group: transient co-transfection of pAAV-RC plasmid, pAAV-Fluc plasmid, and pHelper plasmid; L4-22K group: transient co-transfection of pAAV-RC plasmid, pAAV-Fluc plasmid, pHelper plasmid, and pCMV-22K plasmid; L4-33K group: transient co-transfection of pAAV-RC plasmid, pAAV-Fluc plasmid, pHelper plasmid, and pCMV-33K plasmid; L4-22K+L4-33K group: transient co-transfection of pAAV-RC plasmid, pAAV-Fluc plasmid, pHelper plasmid, pCMV-22K plasmid, and pCMV-33K plasmid; L4-22_33K group: transient co-transfection of pAAV-RC plasmid, pAAV-Fluc plasmid, pHelper plasmid, and pCMV-22_33k plasmid; and NC (negative control) group: transient transfection of empty vector plasmid only.

The results are illustrated in FIG. 1. The titer of the L4-22K group was 8.4E9 vg/mL, which was significantly increased compared with 4.1E9vg/mL of the Empty group (P-value = 0.0114). The titer of the L4-33K group was 1.1E10 vg/mL, which was significantly increased compared with that of the Empty group (P-value = 0.0029). The titer of the L4-22K+L4-33K group was 1.8E10 vg/mL, and the titer of the L4-22_33K group was 2.1E10 vg/mL, with no significant difference in titer between the two groups (P-value = 0.2690). The titer of the L4-22K+L4-33K group was significantly increased compared with that of the L4-22K group (P-value = 0.0308) and also significantly increased compared with that of the L4-33K group (P-value = 0.0478). The titer of the L4-22_33K group was significantly increased compared with that of the L4-22K group (P-value = 0.0068) and also significantly increased compared with that of the L4-33K group (P-value = 0.0090). The titer of the NC group was below the lower limit of detection (5E7 vg/mL).

Based on the above-described results, it can be demonstrated that, when using commonly used adenoviral helper genes (adenovirus E4, E2A, and VA RNA gene sequences carried by the pHelper plasmid) for rAAV vector production, the introduction of L4-22K or L4-33K gene as an additional helper gene significantly improves the rAAV vector yield. More surprisingly, compared with introducing either L4-22K gene or L4-33K gene alone as the additional helper gene, simultaneously introducing both L4-22K gene and L4-33K gene (regardless of whether the two gene sequences are provided separately (L4-22K+L4-33K group) or in an overlapping form as in the L4 region of Ad5 (L4-22_33K group)) further significantly improves the rAAV vector yield, demonstrating that the combined introduction of L4-22K gene and L4-33K gene can produce a significant synergistic effect.

### Example 3: Effect of Introducing L4-22K and/or L4-33K Gene as a Helper Gene on rAAV Vector Production When Using Commonly Used Herpes Simplex Virus Helper Gene

293T cells (ATCC, CRL-3216) were seeded into 6-well plates (Corning 3516) at a density of 8E+05 cells per well in 2 mL of DMEM complete medium. After 24 hours of culture, transfection was performed using the PEI method. 200 µL of transfection reagent was added to each well, with a total plasmid amount of 5 µg, including 0.5 µg of pAAV-EF1a-FLuc-WPRE-HGHpA plasmid (AAV transfer vector carrying a firefly luciferase coding sequence under the control of the EF1a promoter and a WPRE detection sequence, flanked by AAV ITR sequences; Addgene#87951, hereinafter referred to as "pAAV-Fluc plasmid"), 0.5 µg of pAAV-RC plasmid (carrying adenovirus Rep and Cap gene sequences; Novopro, V009666#), 0.3 µg of pCMV-UL5 plasmid, 0.3 µg of pCMV-UL8 plasmid, 0.3 µg of pCMV-UL29 plasmid, and 0.3 µg of pCMV-UL52 plasmid. In addition, the Empty group was supplemented with 0.6 µg of empty vector (pCMV-M1 plasmid)), the L4-22K group was supplemented with 0.6 µg of pCMV-L4-22K plasmid, the L4-33K group was supplemented with 0.6 µg of pCMV-L4-33K plasmid, the L4-22K+L4-33K group was supplemented with a total of 0.6 µg of pCMV-L4-22K and pCMV-L4-33K plasmids (0.3 µg each), and the pCMV-L4-22_33K group was supplemented with 0.3 µg of pCMV-L4-22_33K plasmid. The remaining plasmid amount was made up with the empty vector. The mass ratio of total plasmid amount to PEI was 1:4, and the experiment was independently repeated twice. 48 hours after cell transfection, an equal volume of 1% Tween 20 was added for cell lysis. After the cell lysate was treated with a non-specific nuclease (Yeasen Biotechnology (Shanghai) Co., Ltd., 20156ES60), the adeno-associated virus genome (vg) copy numbers were measured using an ABI QuantStudio 5 real-time fluorescent quantitative PCR system.

In FIG. 2, Empty group: transient co-transfection of pAAV-RC plasmid, pAAV-Fluc plasmid, pCMV-UL5 plasmid, pCMV-UL8 plasmid, pCMV-UL29 plasmid, and pCMV-UL52 plasmid; L4-22K group: transient co-transfection of pAAV-RC plasmid, pAAV-Fluc plasmid, pCMV-UL5 plasmid, pCMV-UL8 plasmid, pCMV-UL29 plasmid, pCMV-UL52 plasmid, and pCMV-22K plasmid; L4-33K group: transient co-transfection of pAAV-RC plasmid, pAAV-Fluc plasmid, pCMV-UL5 plasmid, pCMV-UL8 plasmid, pCMV-UL29 plasmid, pCMV-UL52 plasmid, and pCMV-33K plasmid; L4-22K+L4-33K group: transient co-transfection of pAAV-RC plasmid, pAAV-Fluc plasmid, pCMV-UL5 plasmid, pCMV-UL8 plasmid, pCMV-UL29 plasmid, pCMV-UL52 plasmid, pCMV-22K plasmid, and pCMV-33K plasmid; L4-22_33K group: transient co-transfection of pAAV-RC plasmid, pAAV-Fluc plasmid, pCMV-UL5 plasmid, pCMV-UL8 plasmid, pCMV-UL29 plasmid, pCMV-UL52 plasmid, and pCMV-22_33k plasmid; and NC (negative control) group: transient transfection of empty vector plasmid only.

The results are illustrated in FIG. 2. The titer of the L4-22K group was 1.3E10 vg/mL, which was significantly increased compared with 1.8E9 vg/mL of the Empty group (P-value = 0.0093). The titer of the L4-33K group was 8.9E9 vg/mL, which was also significantly increased compared with that of the Empty group (P-value = 0.0088). The titer of the L4-22K+L4-33K group was 2.0E10 vg/mL, and the titer of the L4-22_33K group was 2.4E10 vg/mL, with no significant difference in titer between the two groups (P-value = 0.1558). The titer of the L4-22K+L4-33K group was significantly increased compared with that of the L4-22K group (P-value = 0.0228) and also significantly increased compared with that of the L4-33K group (P-value = 0.0037). The titer of the L4-22_33K group was significantly increased compared with that of the L4-22K group (P-value = 0.0394) and also significantly increased compared with that of the L4-33K group (P-value = 0.0201). The titer of the NC group was below the lower limit of detection (5E7 vg/mL).

Based on the above-described results, it can be demonstrated that, when using commonly used herpes simplex viral helper gene (UL5, UL8, UL29, UL52) for rAAV vector production, the introduction of L4-22K or L4-33K gene as an additional helper gene significantly improves the rAAV vector yield. More surprisingly, compared with introducing either L4-22K gene or L4-33K gene alone as the additional helper gene, simultaneously introducing both L4-22K gene and L4-33K gene (regardless of whether the two gene sequences are provided separately (L4-22K+L4-33K group) or in an overlapping form as in the L4 region of Ad5 (L4-22_33K group)) further significantly improves the rAAV vector yield, demonstrating that the combined introduction of L4-22K gene and L4-33K gene can produce a significant synergistic effect.

Reference throughout this specification to terms such as "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. A method for producing a recombinant adeno-associated virus vector, the method comprising:
using a nucleic acid sequence encoding adenovirus L4-22K protein and/or a nucleic acid sequence encoding adenovirus L4-33K protein as a helper gene sequence for the production of the recombinant adeno-associated virus vector, without using adenovirus as a helper virus.

2. The method according to claim 1, further comprising using a plurality of additional helper gene sequences.

3. The method according to claim 2, wherein the plurality of additional helper gene sequences comprise one or more nucleic acid sequences from adenovirus, herpes simplex virus, hepatitis C virus, influenza virus, human herpes virus, bocavirus, or human papillomavirus.

4. The method according to claim 2, wherein the plurality of additional helper gene sequences comprise a nucleic acid sequence from adenovirus E1A, E1B, E2A, E4, or VA RNA gene, or a combination thereof.

5. The method according to claim 2, wherein the plurality of additional helper gene sequences comprise a nucleic acid sequence from herpes simplex virus UL5, UL8, UL12, UL29, UL30, UL42, UL52, ICP0, ICP4, ICP22, ICP34.5, or US11 gene, or a combination thereof.

6. The method according to claim 1 or claim 2, comprising introducing the nucleic acid sequence encoding adenovirus L4-22K protein and/or the nucleic acid sequence encoding adenovirus L4-33K protein into a host cell.

7. The method according to claim 6, wherein the host cell is a mammalian cell and optionally selected from HEK293 cells, A549 cells, HeLa cells, HepG2 cells, BHK cells, COS cells, Vero cells, MDCK cells, Per.C6 cells, CAP cells, and a derivative thereof.

8. A recombinant adeno-associated virus vector packaging/production cell for producing a recombinant adeno-associated virus vector without using adenovirus as a helper virus, wherein the packaging/production cell comprises:
adenovirus L4-22K protein or a nucleic acid encoding adenovirus L4-22K protein; and/or
adenovirus L4-33K protein or a nucleic acid encoding adenovirus L4-33K protein.

9. The packaging/production cell according to claim 8, further comprising a nucleic acid sequence from adenovirus E1A, E1B, E2A, E4, or VA RNA gene, or a combination thereof.

10. The packaging/production cell according to claim 8, further comprising a nucleic acid sequence from herpes simplex virus UL5, UL8, UL12, UL29, UL30, UL42, UL52, ICP0, ICP4, ICP22, ICP34.5, or US11 gene, or a combination thereof.

11. Use of adenovirus L4-22K protein or a nucleic acid encoding adenovirus L4-22K protein, and/or adenovirus L4-33K protein or a nucleic acid encoding adenovirus L4-33K protein for the production of a recombinant adeno-associated virus vector without using adenovirus as a helper virus.
